# EUROPEAN PATENT APPLICATION

(11) **EP 3 590 455 A1**
(43) Date of publication of application: **08.01.2020**
(21) Application number: 19183972.9
(22) Date of filing: 02.07.2019
(51) Int. Cl.: A61B 18/18, A61B 18/08, A61B 18/14, A61N 7/02

(54) **BOUNDED ELECTROSURGICAL ENERGY SYSTEMS EMPLOYING ZONE-BASED ENERGY**

(30) Priority: 03.07.2018 US 201816032232
(71) Applicant: Covidien LP, Mansfield, MA 02048 (US)
(72) Inventor: KRIMSKY, William S., Forest Hill, MD Maryland 21050 (US)
(74) Representative: Maschio, Antonio

(57) **Abstract**

A method of treating tissue is provided herein. The method comprises positioning an end effector in a first position in proximity to tissue, wherein the end effector has one or more first thermal elements and one or more second thermal elements and an energy delivery configuration,
activating the one or more first thermal elements, absorbing energy from tissue via the one or more first thermal elements, wherein the energy absorbed is in a first predetermined volume based on the energy delivery configuration, activating the one or more second thermal elements, delivering energy to tissue via the one or more second thermal elements, wherein the energy delivered is in a second predetermined volume based on the energy delivery configuration, and generating a predetermined treatment zone based on the first predetermined volume and the second predetermined volume.

## Description

### BACKGROUND

### Technical Field

The present disclosure relates to systems and methods for treating tissue using multiple energy sources, and more particularly, to the use of multiple energy sources to generate and deliver zone-based energy therapies and treatments to tissue.

### Background of Related Art

Treatment of certain diseases requires the destruction of malignant tissue growths, such as tumors. Various radiation techniques can be used to heat and destroy tumor cells. Treatment may involve inserting probes into tissues where tumors have been identified. Once the probes are positioned, energy is passed through the probes and into surrounding tissue, or in the case of cooling; energy is passed from the tissue into the probes.

In the treatment of diseases such as cancer, certain types of tumor cells have been found to denature at elevated temperatures that are slightly lower than temperatures normally injurious to healthy cells. Known treatment methods, such as hyperthermia therapy, heat diseased cells to temperatures above 41° C while maintaining other healthy cells below the temperature at which irreversible cell destruction occurs.

The heating of tissue for thermal treatment can be accomplished through a variety of approaches, including conduction of heat from an applied surface or element of the probe, or, in the case of microwave treatment, by dielectric relaxation of water molecules within an antennas electromagnetic field. In addition to heating of tissue, cryoablation may also be utilized. Cryoablation is a therapy that uses that removal of heat from tissue, to treat various regions of interest within tissue. In most cryoablation procedures, a pressurized refrigerant is circulated within the tip of a cryoablation catheter, where the refrigerant expands and absorbs heat from surrounding tissue.

The treatment zone created can be broken down into two components: an active treatment zone and a passive treatment zone. The active treatment zone is generally closest in proximity to a heating element or a radiating portion of a surgical probe and encompasses the volume of tissue which is subjected to energy absorption high enough to assure thermal tissue treatment and/or destruction at a given application time in all but areas of very rapidly flowing fluids, such as around and within large blood vessels or airways. Similarly, for cryoablation the active treatment zone encompasses the volume of tissue which has its temperature reduced sufficiently to destroy or damage the tissue.

The passive treatment zone generally surrounds the active treatment zone and encompasses the volume of tissue or other biological material which experiences a lower intensity of energy absorption and/or temperature reduction. In some instances physiological cooling may counter heating therefore not allow for sufficient heating to occur within the passive zone to treat tissue, in particular those tissues in close proximity to a major blood vessel.

Because of the small temperature difference between the temperature required for treating malignant cells and the temperature normally injurious to healthy cells, a known heating pattern and precise temperature control is needed to ensure predictable temperature distribution to eradicate the tumor cells while minimizing the damage to surrounding normal tissue. Due to the varying scenarios of tissue and biological material in need of treatment, there exists further need for the systems and methods to enable the generation of various shapes and volumes of active and passive treatment zones.

### SUMMARY

In accordance with the present disclosure, a method of treating tissue is provided. The method includes positioning an end effector in a first position in proximity to tissue, wherein the end effector can have one or more first thermal elements and one or more second thermal elements, or insulators, and an energy delivery configuration, activating the one or more first thermal elements, absorbing energy from tissue and or insulating the tissue via the one or more first thermal elements, wherein the energy absorbed is in a first predetermined volume based on the energy delivery configuration, activating the one or more second thermal elements, delivering energy to tissue via the one or more second thermal elements, wherein the energy delivered is in a second predetermined volume based on the energy delivery configuration, and generating a predetermined treatment zone based on the first predetermined volume and the second predetermined volume.

In another embodiment of the present disclosure, the second predetermined volume includes at least a portion of the first predetermined volume. In yet another embodiment of the present disclosure, the first predetermined volume is a cooling zone located in proximity to one or more first thermal elements and the second predetermined volume is an active heated treatment zone located in proximity to the one or more second thermal elements.

In a further aspect of the present disclosure, absorbing energy from tissue is done for a first predetermined interval of time and delivering energy to tissue is done for a second predetermined interval of time. In yet another aspect of the present disclosure, absorbing energy from tissue and delivering energy to tissue is done simultaneously. In a further aspect, the method of the present disclosure further includes alternating between absorbing energy from tissue and delivering energy to tissue.

In another aspect of the present disclosure, the energy delivery configuration is a cylindrical configuration wherein the one or more first thermal elements is a cylindrical tubular member containing a passageway and the one or more second thermal elements is a cylindrical member located inside the passageway. In yet another aspect of the present disclosure, the one or more second thermal elements is configured to be deployed outside of the passageway.

In another embodiment of the present disclosure, the energy delivery configuration is a rectangular configuration of stacked rectangular thermal elements alternating between the one or more first thermal elements and the one or more second thermal elements. In a further aspect of the present disclosure, the energy deliver configuration bounds a thermal spread of the energy delivered to tissue.

In another embodiment of the present disclosure, the method further includes repositioning the end effector in one or more other positions in proximity to tissue, wherein the one or more other positions and the first position are different, absorbing energy from tissue at the one or more other positions, delivering energy to tissue at the one or more other positions, and creating another predetermined treatment zone based on the energy delivered at the first position and one or more other positions.

According to another aspect of the present disclosure, a tissue treatment system is provided. The system includes an end effector assembly, including one or more first thermal elements and one or more second thermal elements, wherein the end effector assembly has an energy delivery configuration and is configured to deliver energy to tissue and absorb energy from tissue in a predetermined treatment zone based on the energy delivery configuration, a generator coupled to the end effector assembly and configured to supply energy to the one or more first thermal elements or one or more second thermal elements, and a coolant source coupled to the end effector and configured to supply a coolant fluid to the one or more first thermal elements or one or more second thermal elements.

### BRIEF DESCRIPTION OF THE DRAWINGS

Various aspects and features of the present disclosure are described herein with reference to the drawings wherein:
FIG. 1 is a perspective view of an illustrative embodiment of a treatment system, in accordance with embodiments of present disclosure;
FIG. 2 is a partial, perspective view of a first configuration of a treatment probe of the treatment system of FIG. 1, in accordance with an embodiment of the present disclosure;
FIG. 3A is a partial, perspective view of a second configuration of a distal portion of a treatment probe of the treatment system of FIG. 1 in a retracted position, in accordance with an embodiment of the present disclosure;
FIG. 3B is a partial, perspective view of the distal portion of the treatment probe of FIG. 3A in a deployed position, in accordance with an embodiment of the present disclosure;
FIG. 4 is a partial, perspective view of a third configuration of a distal portion of a treatment probe of FIG. 1, in accordance with an embodiment of the present disclosure;
FIG. 5 is a partial, perspective view of a fourth configuration of a distal portion of a treatment probe of FIG. 1, in accordance with an embodiment of the present disclosure; and
FIGs. 6A-6H are partial, perspective views of the treatment probe configurations of FIGs. 3A, 3B, 4, and 5 inserted into a treatment site, activated, and a generated treatment zone, in accordance with embodiments of the present disclosure.

The figures depict particular embodiments of the present disclosure for purposes of illustration only. One skilled in the art will readily recognize from the following discussion that alternative embodiments of the structures and methods illustrated herein may be employed without departing from the principles described herein.

### DETAILED DESCRIPTION

The present disclosure relates to systems and methods for treating tissue by using a treatment probe capable of simultaneously and/or alternately applying heating and cooling to tissue surrounding the treatment probe. In embodiments, the heating may be performed by radiating energy outward via microwave radiation, and the cooling may be applied by absorbing energy from surrounding tissue via active cooling and/or emitting cooling energy from the treatment probe. Insulating material may be provided to prevent non-target areas from receiving energy and/or to prevent heating and cooling elements from impacting each other. Cooling tissue immediately surrounding the treatment probe while radiating microwave energy from the treatment probe may enhance the overall heating pattern and assist with regulating both the treatment temperature and the treatment zone. Particular embodiments of the present disclosure are described herein below with reference to the accompanying drawings; however, it is to be understood that the disclosed embodiments are merely exemplary and can be applied to any energy source and its counterpoint such as radiation, etc. Therefore, specific structural and functional details described herein are not to be interpreted as limiting, but merely as a basis for the claims and as a representative basis for teaching one skilled in the art to variously employ the presently disclosed concepts in any appropriately detailed structure.

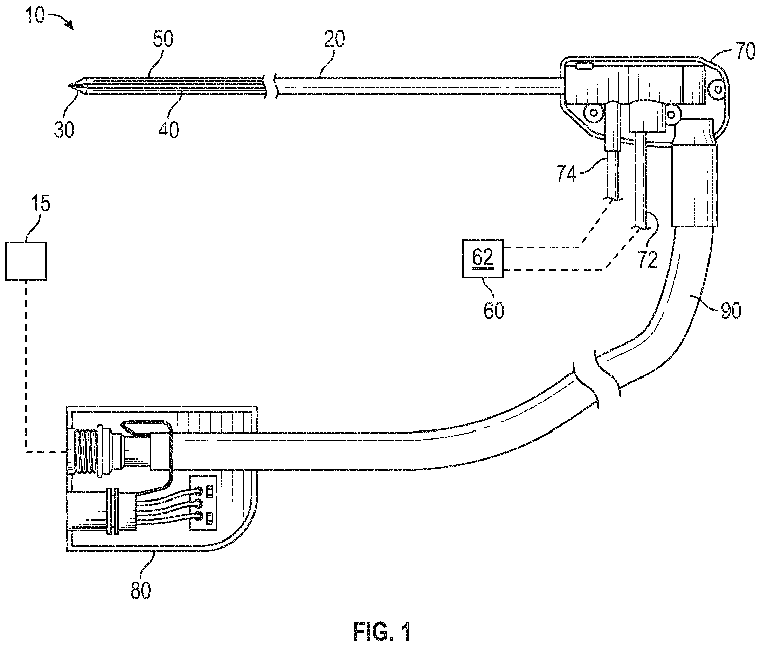

With reference to FIG. 1, a treatment system 10 is provided for use with the methods of treating tissue described in detail below. Treatment system 10 generally includes a generator 15 and a treatment probe 20. Treatment probe 20 includes a distal portion 30 including at least one heating portion 40 and at least one cooling portion 50. In embodiments, treatment system 10 may further include a coolant source 60.

Generator 15 is configured to provide energy to probe 20, thereby generating microwave radiation which may be transferred to tissue proximate treatment probe 20. In other embodiments, generator 15 may generate any suitable type of energy, for example, radio frequency (RF) electrosurgical energy, therapeutic ultrasound energy, and/or thermal energy via resistive elements.

Heating portion 40 of probe 20 may also be referred to as a first thermal element and cooling portion 50 as a second thermal element. Although the first thermal element and second thermal element are referenced with respect to heating portion 40 and cooling portion 50, respectively, it is contemplated that the first thermal element and the second thermal element may be used interchangeably with each of heating portion 40 and cooling portion 50. Thus, in some embodiments, the first thermal element may be cooling portion 50 and the second thermal element may be heating portion 40. Heating portion 40 is configured to radiate energy provided by generator 15 and cooling portion 50 is configured to utilize a coolant fluid 62 (e.g., liquefied CO₂) from coolant source 60 to absorb energy and provide therapeutic cooling or treatment (e.g., cryoablation) to tissue proximate an exterior surface of distal portion 30. Although distal portion 30 is illustrated in a conical configuration, in other embodiments distal portion 30 may include various other configurations, as further described hereinbelow. Further, those skilled in the art will appreciate that embodiments of heating portion 40 and cooling portion 50 are not limited to the examples mentioned above, and that heating portion 40 could be or include any heating method or modality, and likewise cooling portion 50 could be or include any cooling method or modality.

Probe 20 is coupled to a handle assembly 70. Handle assembly 70 includes an inlet fluid port 72 and an outlet fluid port 74. In one embodiment fluid ports 72 and 74 are both in fluid communication with coolant source 60. Thus, coolant fluid 62 may circulate from coolant source 60, through port 72 and vaporize into a coolant vapor 64, as described herein, within areas of cooling portion 50 of probe 20 to cool tissue during use. The coolant vapor 64 may be drawn back to coolant source 60 via port 74, where it may be compressed and condensed and re-circulated through the system in accordance with common vapor recovery refrigeration systems. Alternatively, coolant vapor 64 may be vented to the atmosphere. Further, in some embodiments, cooling portion 50 may, instead of using a circulated coolant fluid 62, include one or more one thermoelectric devices within cooling portion 50 which are configured to cryogenically cool the surface of cooling portion 50 via the Peltier effect.

Probe 20 and generator 15 are coupled to one another via a connector assembly 80 and a cable assembly 90. Connector assembly 80 is a cable connector suitable to operably connect cable assembly 90 to generator 15. Connector assembly 80 may house a memory (e.g., an EEPROM) storing a variety of information regarding various components of system 10.

Cable assembly 90 interconnects connector assembly 80 and probe 20 to allow for the transfer of energy from generator 15 to heating portion 40. Cable assembly 90 may be any suitable, flexible transmission line, such as a coaxial cable, including an inner conductor, a dielectric material coaxially surrounding the inner conductor, and an outer conductor coaxially surrounding the dielectric material. Cable assembly 90 may be provided with an outer coating or sleeve disposed about the outer insulator. The sleeve may be formed of any suitable insulative material, and may be applied by any suitable method, e.g., heat shrinking, over-molding, coating, spraying, dipping, powder coating, and/or film deposition. For a more detailed description of a microwave ablation system, reference may be made to U.S. Patent Application Publication No. 2014/0276033, entitled "MICROWAVE ENERGY-DEVICE AND SYSTEM," filed on March 15, 2013, by Brannan et al., the entire contents of which are incorporated herein by reference.

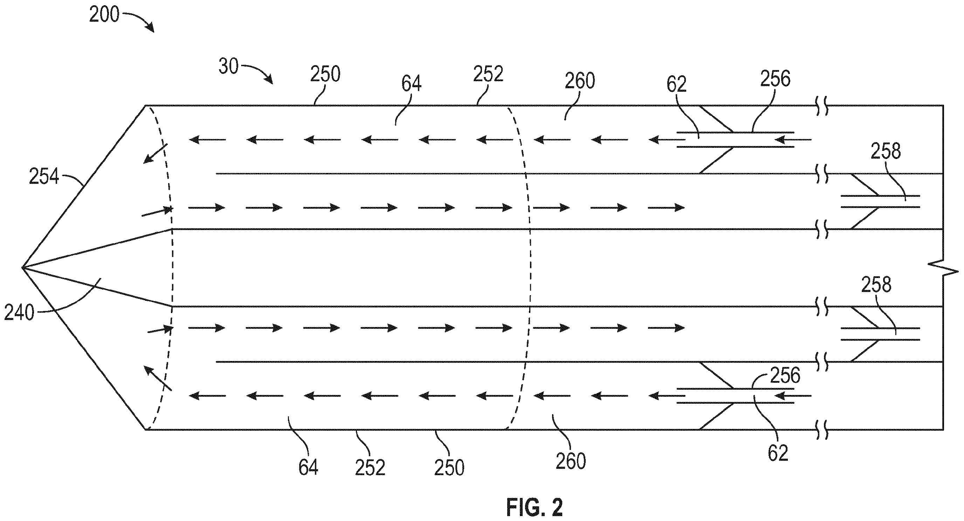

Referring now to FIG. 2, a cross-section of a first configuration 200 of distal portion 30 of probe 20 is shown in greater detail, in accordance with an embodiment of the present disclosure. As shown in FIG. 2, distal portion 30 assembled according to configuration 200 includes a heating portion 240 and a cooling portion 250. Cooling portion 250 is formed as two semi-cylindrical hollow members having a surface 252, a distal face 254, inlet tubes 256, outlet tubes 258, and coolant channels 260. Coolant fluid 62 is supplied via the inlet tubes to the coolant channel 260 where, due to the pressure differential between the inlet tube 256 and the coolant channel 260, coolant fluid 62 flashes to coolant vapor 64. In principal, this is similar to the effect experienced at an orifice or thermal expansion valve in a refrigeration cycle. Coolant fluid 62 continues to vaporize and coolant vapor 64 continues to expand such that only vapor enters the outlet tubes 258. In this manner, surface 252 is cooled allowing cooling portion 250 to absorb thermal energy from surrounding tissue, and thereby cool the tissue proximate surface 252. In some instances, in addition to having absorbed the energy associated with vaporization (e.g., the latent heat of vaporization) the vapor may continue to absorb heat (e.g., sensible heat) prior to entry into the outlet tubes 258. Included within a central portion of distal portion 30 is heating portion 240. That is, heating portion 240 extends longitudinally through and along a central axis of distal portion 30. Heating portion 240 is supplied with energy by generator 15 and is configured, in one embodiment, to emit microwave radiation from heating portion 240 through cooling portion 250 and to surrounding tissue, thereby heating and treating tissue surrounding distal portion 30.

Although shown as two distinct members, it is contemplated that each of the two semi-cylindrical hollow members of cooling portion 250 join to fully surround heating portion 240. In other embodiments, a single cooling portion 250 is utilized with a single inlet tube 256 and a single outlet tube 258.

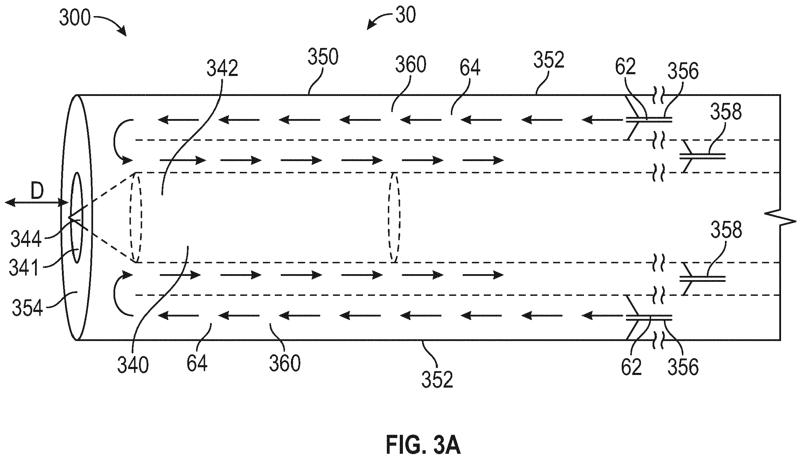

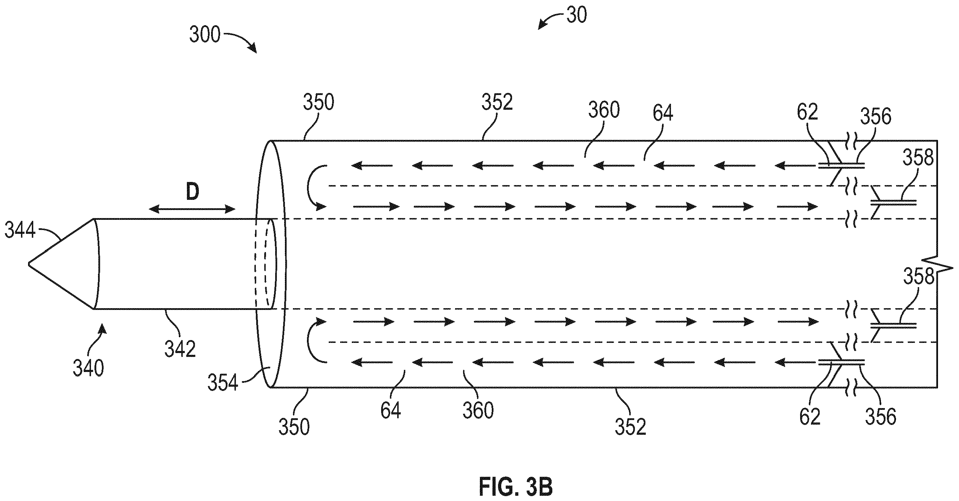

Turning now to FIGS. 3A and 3B, a cross-section of a second configuration 300 of distal portion 30 of probe 20 is shown in greater detail, in accordance with an embodiment of the present disclosure. Similar to configuration 200 of FIG. 2, distal portion 30 assembled according to configuration 300 includes a heating portion 340 positioned inside of a passageway 341 formed along a central axis of distal portion 30 surrounded by a cooling portion 350. Heating portion 340 further includes a surface 342 and a distal face 344. It is contemplated that surface 342 of heating portion 340 and the inner surface of cooling portion 350 are insulated in order to minimize thermal transfer between heating portion 340 and cooling portion 350 during use. As illustrated in FIG. 3A, cooling portion 350 is a hollow tubular member with a surface 352 and distal face 354, and that cooling portion 350 surrounds passageway 341. Heating portion 340 is a cylindrical member disposed within passageway 341 and capable of being moved in a direction "D" from a first position, located within passageway 341 (FIG. 3A) to a second position, located at least partially outside and distal of passageway 341 (FIG. 3B).

As further shown in FIG. 3A, located at a proximal end of distal portion 30 are inlet tubes 356 and outlet tubes 358 which allow the transfer of coolant fluid 62 to and from cooling source 60 and through coolant channel 360 of cooling portion 350. It is contemplated that heating portion 340 and cooling portion 350 may be switched between the hollow tubular member that is shown as cooling portion 350 and the cylindrical member that is shown as heating portion 340. Thus, in some embodiments, the hollow tubular member may be heating portion 340 and the cylindrical member may be cooling portion 350.

Turning now to FIG. 3B, a cross-section of configuration 300 of distal portion 30 of probe 20 is shown in a deployed position. Heating portion 340 is illustrated in a deployed position at least partially located outside and distal of passageway 341. Based on the deployed position, heating portion 340 is capable of being activated and generating microwave energy to be absorbed by tissue in closer proximity to surface 342 of heating portion 340, distal of passageway 341, while cooling portion 350 may be activated alternatively or simultaneously, thereby creating overlapping heating and cooling zones, as described hereinbelow.

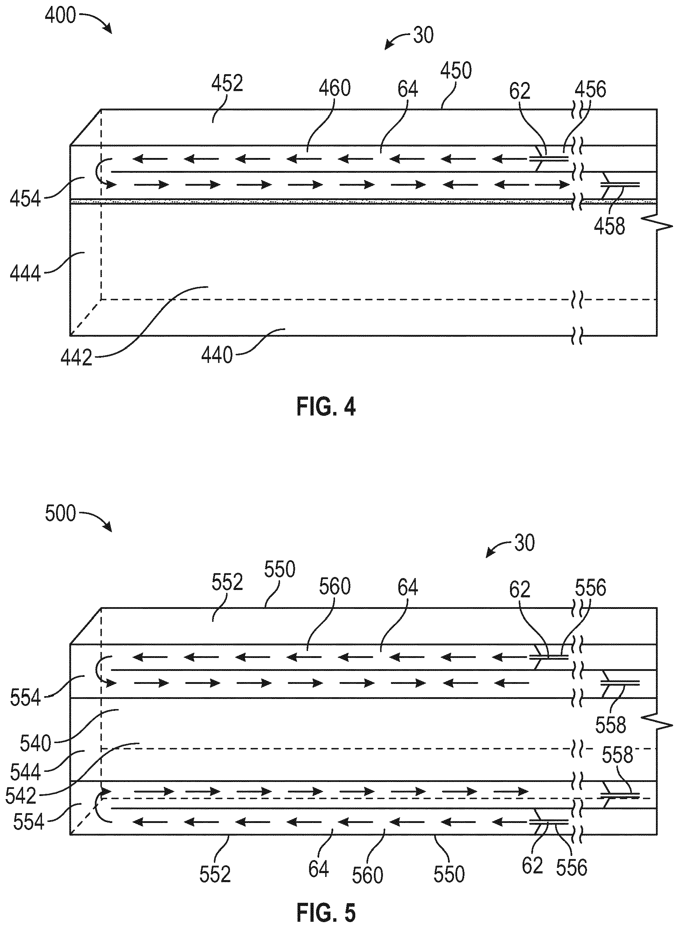

Referring now to FIGS. 4 and 5, cross-sections of stacked rectangular configurations 400 and 500 of distal portion 30 of probe 20 are illustrated. In an embodiment, a distal portion 30 assembled according to configuration 400 includes a rectangular cuboid heating portion 440 and rectangular cuboid cooling portion 450. Heating portion 440 includes surface 442 and distal face 444. Cooling portion 450 includes surface 452, distal face 454, inlet tube 456, outlet tube 458, and coolant channel 460 where coolant fluid 62 flashes into coolant vapor 64. In configuration 400, one heating portion 440 is stacked with one cooling portion 450.

In another embodiment, a distal portion 30 assembled according to configuration 500 includes a single rectangular cuboid heating portion 540 positioned between two rectangular cuboid cooling portions 550. Heating portion 540 includes surface 542 and distal face 544. Cooling portion 550 includes surface 552, distal face 554, inlet tube 556, outlet tube 558, and coolant channel 560 where coolant fluid 62 flashes into coolant vapor 64 similar to configurations 200-400 described above. Although configuration 500 is shown as three distinct rectangular elements, it is contemplated that configuration 500 may include four thermal elements or five thermal elements or the like. In further embodiments, distal faces 444, 454, 544, and 554, of configurations 400 and 500, may include rounded and/or conical corners. Embodiments include systems that oscillate between heating and cooling such that energy is bounded. Shielding may be provided on the external source(s) to prevent leakage, whereby the energy is transmitted at a treatment dose between the two counterpointed sources. Based on the various configurations, distal portion 30 is capable of generating specific thermal spreads of various shapes and sizes. Distal portions 30 arranged according to the above-described configurations, as well as other configurations having multiple thermal elements, may generate thermal zones of various shapes and sizes, and the shapes and sizes may be specifically determined according to the configuration used. Further, due to the heating and cooling energy being opposites, the thermal effect generated by applying simultaneous and/or alternating heating and cooling energy to tissue surrounding probe 20 may be attenuated and/or magnified, depending on the application. For example, heating energy may be applied to tissue as the "treatment," which is then bounded by cooling energy such that the intensity and effect of the zone of tissue heated by the heating energy between the "poles" of heating and cooling energy may be magnified. Additionally, there will be a treatment effect from the poles that can also be enhanced and/or attenuated. These poles may also be insulated such that injury to tissue is limited to the targeted treatment area. As will be appreciated by those skilled in the art, other forms of energy, such as microwave energy or radiation, may also be used as the "treatment" and the bounding (cooling) components may be something as simple as insulators or reflector of the primary energy source or the counterpoint to that source such that treatment can be enhanced in both the heating and cooling zones.

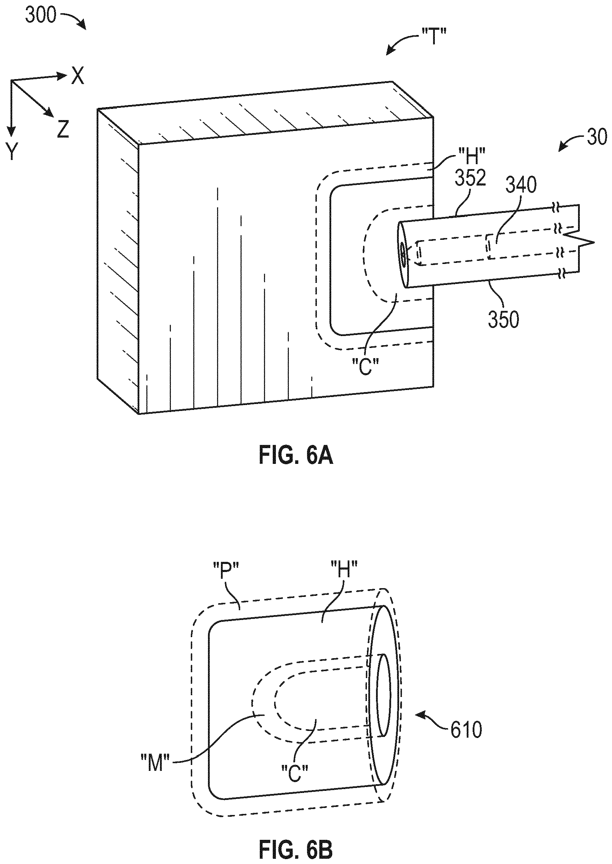

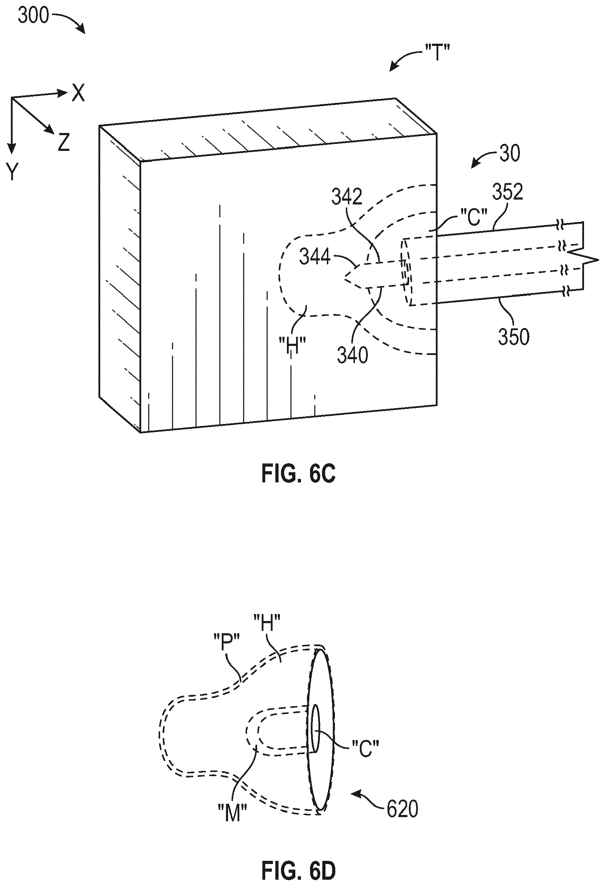

With reference to FIGS. 6A-6H, probe 20 is shown inserted into a treatment site "T,' and the generation of specific treatment zones "Z" within treatment site "T" are illustrated. FIGS. 6A and 6C illustrate treatment system 10 in use with distal portion 30 in configuration 300 as shown in FIGS. 3A and 3B, respectively. During a procedure, for example, a minimally invasive procedure such as a laparoscopic, endobronchial, or percutaneous procedure, access is gained to a surgical treatment site "T" within a patient by inserting probe 20 into the patient's body. For laparoscopic or endoscopic procedures, a cannula, trocar, or any suitable access port, having a stylet at its distal end may be used to access a body cavity of a patient. In the example of a laparoscopic procedure, probe 20 is inserted within the cannula (not shown) that extends into the surgical site. Distal portion 30 is placed in suitable position and proximity within a specific area of treatment site "T," for example, next to or in contact therewith. In FIGS. 6A-6H, distal portion 30 including heating portion 40 and cooling portion 50 are used to generate a treatment zone having a heated zone "H" and a cooling zone "C." By applying cooling and heating energy either simultaneously or alternately to tissue and other biological material in the treatment zone, treatment zones of various shapes and sizes may be generated, as illustrated in FIGS. 6A-6H. For example, in the embodiment shown in FIG. 6A, distal portion 30, assembled according to configuration 300, is placed in a suitable position within treatment site "T," with heating portion 340 in a retracted position. Heating portion 340 and cooling portion 350 are shown inserted into treatment site "T" with surface 352 of cooling portion 350 in contact with the tissue and other biological material of treatment site "T." Cooling portion 350 may be held in a suitable position while activated to generate a cooling zone "C." Cooling portion 350 reduces the temperature of tissue and other biological material in close proximity to surface 352, thus enabling a clinician to regulate the temperature of the treatment zone and thus prevent tissue closer to probe 20 from being heated to too high a temperature while tissue further away from probe 20 is being heated. In some embodiments, probe 20 may further include one or more temperature sensors (not shown) along the length of distal portion 30, and cooling portion 350 may be selectively and/or automatically activated when it is determined that the temperature of tissue proximate distal portion 30 reaches a threshold temperature. For example, threshold temperature may be 50 or 60 degrees centigrade, and thus, if it is determined that the temperature of tissue proximate distal portion 30 reaches 50 or 60 degrees centigrade, cooling portion 350 may be activated, or provision of cooling fluid 62 to cooling portion 350 may be enhanced, to prevent the temperature of the tissue proximate distal portion 30 from exceeding the threshold temperature. The threshold temperature may be predetermined and/or adjusted by the clinician prior to and/or during the treatment procedure.

In addition to regulating the temperature of tissue proximate distal portion 30, cooling portion 350 may also be selectively activated to regulate and/or control the size and shape of the treatment zone. The cooling temperature may be selected and or adjusted at any time depending on purpose. For example, containment within a target area may only require a minimally sufficient cooling temperature to prevent leakage of the heat-related thermal energy. Additionally for example, cooling temperature for treating tissue requirements may also depend on the patient, the treatment, and various other factors, and may for example be sufficiently low to reduce tissue temperatures to about minus 10 degrees centigrade

As shown in FIG. 6A, cooling portion 350 is activated and generating cooling zone "C" either prior to, during, and/or after heating portion 340 is activated to generate a specific shape and configuration of an heated treatment zone "H." Thus, cooling portion 350 may be used to both limit damage to tissue proximate distal portion 30, and limit the size of a passive treatment zone "P" caused by passive heating (primarily through conduction) of tissue beyond the heated treatment zone "H." As illustrated in FIG. 6A and described herein, a clinician may activate cooling portion 350, causing coolant fluid 62 to flow to coolant channel 260 and flash to coolant vapor 64, thereby reducing the temperature of surface 352 of cooling portion 350, and, in turn, reducing the temperature of adjacent tissue within treating site "T," shown as the cooling zone "C." Continued and prolonged activation of cooling portion 350 for a predetermined amount of time increases the volume of cooling zone "C." The predetermined amount of time, which corresponds to a specific volume, is dependent on the type of tissue, tumor, and/or other biological material present in treatment site "T." Additionally, the time may be dependent upon the purpose of the cooling: if it is to maintain the viability of tissue, the application of cooling energy may be shorter to prevent cooling to the point of tissue destruction. Alternatively, if the purpose is to create a heat sink effectively limiting the size of the passive treatment zone "P" (for example to avoid damaging certain tissues) the application of the cooling energy may be of longer duration. As noted above, cooling portion 350 may be activated selectively and/or automatically based on temperature sensors included in distal portion 30.

As shown in FIG. 6A, cooling zone "C" is illustrated as a cylindrical volume of cooled tissue radiating outwards from distal portion 30. Due to the temperature transfer between volumes of tissue closer to and farther from cooling portion 350, those volumes of tissue closer to surface 352 of cooling portion 350 may be maintained at temperatures lower than those areas farther from surface 352 of cooling portion 350. Therefore, although shown as uniform, cooling zone "C" zone may include a gradient of temperatures being cooler closer to surface 352 and becoming progressively hotter as the distance to surface 352 increases. Based on the disclosure herein, following the application of heating energy via heating portion 340 to generate heated treatment zone "H," the temperature of the tissue in cooling zone "C" remain lower than those of volumes outside of cooling zone "C." Thus, during the activation of heating portion 340, tissue adjacent to surface 352 of cooling portion 350 may remain viable despite the creation of the heated treatment zone "H" where the tissue reaches higher temperatures, thus denaturing and/or destroying the tissue. The volumes of tissue that are cooled in cooling zone "C" rely in part on conduction to transfer heat from the warmer tissues to cooler tissues and, in turn, to cooling portion 350. Upon activation of heating portion 340, heating energy, such as microwave energy, increases the temperature of the surrounding tissue. The tissue adjacent to heating portion 340 heated by the heating energy increases in temperature to form the heated treatment zone "H." In addition, due to conduction, the tissue adjacent to heated treatment zone "H" also increases in temperature. Cooling zone "C" creates a heat sink which is utilized to draw heat from the outer edges of heated treatment zone "H," and/or passive treatment zone "P," as detailed in the description of FIG. 6B. As heat is absorbed by the surrounding tissue in an effort to return the body to normal temperature, the heat from the heated treatment zone "H" is drawn toward cooler tissues, including the cooling zone "C." In addition, the tissue which has had its temperature reduced within the cooling zone "C," increases in temperature at a slower rate than the tissue which was not cooled by cooling portion 350, and, as shown in FIG. 6A, is farther from surface 352 of cooling portion 350.

As shown in FIG. 6B, a 3D model 610 of heated treatment zone "H," a margin zone "M," cooling zone "C," and passive treatment zone "P" is illustrated, showing the specific treatment shape and configuration of the treatment zone generated based on the cooling and heating energy applied to treatment site "T" by distal portion 30. As shown in model 610, margin zone "M" is located between heated treatment zone "H" and cooling zone "C," and acts as a transition zone between the temperatures of the heated treatment zone "H" and cooling zone "C." Thus, heated treatment zone "H," may be utilized to ablate and/or treat tissue located away from distal portion 30, while cooling zone "C" acts as a buffer between surface 352 of cooling portion 350 and heated treatment zone "H," while also limiting the spread of the passive treatment zone "P," by drawing in heat from heated treatment zone "H."

Referring now to FIG. 6C, configuration 300 is illustrated with heating portion 340 deployed at least partially beyond distal face 354 of cooling portion 350. With heating portion 340 deployed, heating portion 340 may be activated to radiate microwave energy from surface 342 while cooling portion 350 is activated or deactivated. As illustrated in FIG. 6C, heating portion 340 is disposed in contact with tissue and/or other biological material of treatment site "T." Once activated, the temperature of tissue and/or biological material in close proximity to surface 342 of heating portion 340 increases. However, due to the prior, concurrent, and/or subsequent activation of cooling portion 350, tissue and other biological material proximate the cooling portion 350 increases in temperature at a slower rate than the tissue and other biological material which was not cooled (e.g., located farther from cooling portion 350).

As illustrated in FIG. 6D, a 3D model 620 of heated treatment zone "H," cooling zone "C," margin zone "M," and passive treatment zone "P" is illustrated, showing a specific treatment shape and configuration of a treatment zone that may be generated based on the cooling and heating energy applied to treatment site "T" by distal portion 30 when heating portion 340 is deployed. Thus, as shown in FIG. 6D, by using configuration 300, tissue and other biological material located in cooling zone "C" remains at lower temperatures while tissue and other biological material located in heating treatment zone "H" are heated to higher temperatures. Additionally, passive treatment zone "P" is drawn inward due to the heat sink created by cooling zone "C."

In some embodiments, application of cooling and heating energy to tissue may be alternated and/or cycled. The cycling of heating and cooling energy applications are part of what may enable the projection of the heating effects of heating portion 340 beyond tissue that is to remain viable through the application of cooling energy via cooling portion 350. These cycles may be very short and rely on a compounding effect of energy application to generate sufficient heat within heated treatment zone "H," while the effects of the heating are negated in the portion of the tissue that are cooled, before, during, and/or after the application of heating energy.

In embodiments, heating portion 340 can be deployed or retracted from cooling portion 350 and both cooling portion 350 and heating portion 340 can be activated simultaneously to generate heated treatment zone "H" and cooling zone "C" of a specific shape and/or size. For instance, in one example, where probe 20 is inserted via an endobronchial procedure and into a patient's airways, it may be necessary to heat and treat areas of tissue outside of the airway without treating, or otherwise harming the airway walls. In this example, distal portion 30 of probe 20, assembled according to configurations 200 or 300, can be in inserted into the patient's airways and cooling portion 350 can be activated to cool the airway walls while heating portion 340 is activated simultaneously to heat tissue away from distal portion 30, thereby heating and treating tissues and regions outside of the airway without treating the airway wall adjacent to distal portion 30. In a further embodiment, heating portion 340 may be deployed from cooling portion 350 and each may be activated in pulses for predetermined periods, thereby generating another specific shape of heated treatment zone "H," cooling zone "C," and passive treatment zone "P."

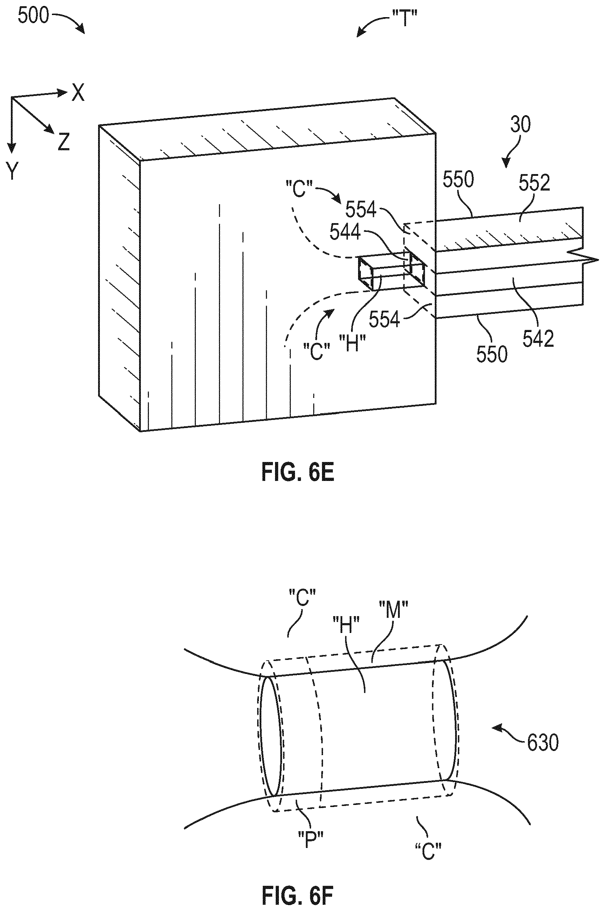

As illustrated in FIG. 6E, distal portion 30 is activated in close proximity to treatment site "T," thereby generating heated treatment zone "H" and cooling zone "C" in a specific pattern based on configuration 500 (FIG. 5). As cooling portions 550 are activated, tissue adjacent to cooling portions 550 are cooled due to the energy being absorbed by cooling portions 550. In configuration 500, the two cooling portions 550, once activated, are configured to reduce the temperature of tissue and biological material in proximity to surfaces 552 and distal face 554 and to adjacent tissue due to conduction. As heating portion 540 is activated, either alternatively or simultaneously with cooling portion 550, the volume of tissue that is treated is in heated treatment zone "H" is bounded by the regions of cooled tissue or other biological material created by cooling zone "C." Thus, configuration 500, once activated, bounds the spread of heat which is radiated from heating portion 540.

FIG. 6F shows a 3D model 630 of heated treatment zone "H," cooling zone "C," and margin "M" between the heated treatment zone "H" and cooling zone "C." In the margin "M," the temperature of the tissue will have a gradient and may be heated sufficiently to denature the tissue, particularly closer to the heated treatment zone "H." As can be imagined, during treatment, management of the margin "M," either to ensure sufficient tissue has been treated or to ensure that certain tissues are maintained as viable, can be achieved by managing the energy, either heating or cooling, the cycle times, and other factors. Further, in some instances it may be desirable to limit the margin "M" to as small a size as possible to provide greater clarity on the tissue that has been treated and tissue that remains viable, to thereby limit the portions of tissue of which the degree of treatment is uncertain.

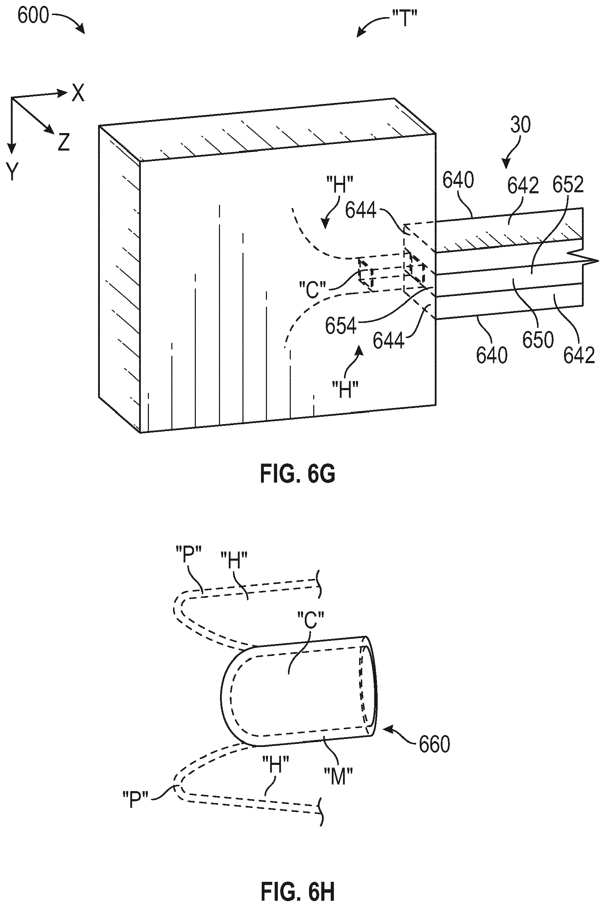

Referring now to FIG. 6G, distal portion 30 assembled according to a configuration 600 similar to that of configuration 500 is illustrated. The configuration shown in FIG. 6G includes two heating portions 640 and one cooling portion 650 in proximity to treatment site "T." Cooling portion 650 is bounded by both heating portions 640. Similar to FIG. 6E, once activated, distal portion 30 generates cooling zone "C" and heated treatment zone "H" in a specific pattern based on the configuration. Cooling portion 650, bounded by heating portions 640, generates a treatment zone shape where heated treatment zone "H" extends from surface 642 and distal face 644 of heating portion 640 and has a middle volume which includes cooling zone "C" in closer proximity to surface 652 and distal face 655 of cooling portion 650. Thus, in the example illustrated in FIG. 6G, distal portion 30 assembled according to configuration 600 allows distal portion 30 to be placed in close proximity to tissue and/or other biological material and activated while preventing thermal heating of a margin zone "M." As shown in FIG. 6H, 3D model 660 illustrates heated treatment zone "H," cooling zone "C," and a margin "M" between the heated treatment zone "H" and cooling zone "C."

In some embodiments, other types of energy (e.g., Radiofrequency, therapeutic ultrasound, or resistive heating) may be emitted from distal portion 30 to heat the tissue and other biological material of treatment site "T." Additionally, it is contemplated that during a surgical procedure utilizing probe 20, one or more visualization techniques including ultrasound imaging, computed tomography (CT), fluoroscopy, and or direct visualization may be used to accurately guide the probes 12 into the area of treatment site "T."

With respect to the generation of heated treatment zone "H" and cooling zone "C," it is further contemplated that distal portion 30 may be re-positioned and relocated in one or more additional suitable positions within treatment site "T," thereby creating overlapping heated treatment zone "H" and cooling zone "C." For example, a torus shape may be generated via generation of cooling zones "C" and heating zones "H" and repositioning of distal portion 30. In the illustration shown in FIG. 6G, a clinician is able to reposition distal portion 30 in positions within a single plane, such as the x-y plane. The clinician may position distal faces 644, 654 of distal portion 30 at a specific location within treatment zone "T" and reposition distal portion 30 around that specific location. In this example, a clinician may perform the following steps: (1) position distal portion 30 in a negative x-direction, as shown in FIG. 6G, and activate both heating portion 640 and cooling portion 650; (2) reposition distal portion 30 in a positive y-direction at the specific location, and activate both heating portion 640 and cooling portion 650; (3) reposition distal portion 30 in a positive x-direction at the specific location, and activate both heating portion 640 and cooling portion 650; and (4) reposition distal portion 30 in a negative y-direction at the specific location, and activate both heating portion 640 and cooling portion 650. Through this repositioning a clinician is able to generate a specific torus shape of a heated treatment zone "H," while creating an interior volume of cooling zone "C." Thus, a clinician is capable of placing distal portion 30 in close proximity to a region of interest while treating the areas surrounding the region of interest without treating the region of interest.

Although the embodiments described in the descriptions of FIGS. 6A-6H detail the use of heating portions 340, 540, and 640 to generate a heated treatment zone "H" while cooling portions 350, 550, and 650 generate a cooling zone "C," in further embodiments, where cryoablation is utilized to treat tissue, cooling portions 350, 550, and 650, may be utilized to generate a cooled treatment zone to cryoablate tissue while heating portions 340, 540, and 640 may be utilized to generate a heating zone to minimize the decrease in temperature around the cooled treatment zone. The above examples should not be construed as limiting, but merely as exemplifications of particular embodiments. Those skilled in the art will envision other modifications within the scope of the claims appended hereto.

It is envisioned that each of the disclosed methods of treating tissue may be performed under CT, MRI, direct thermometry using MRI or CT, or ultrasound for conformational density measurements. In some embodiments, the effects of heat or ice on tissue may be used as visual cues to determine when to switch between usage of cooling portion 50, and heating portion 40 during the surgical procedure.

As it is used in this disclosure, "microwave" generally refers to electromagnetic waves in the frequency range of 300 megahertz (MHz) (3 x 10⁸ cycles/second) to 300 gigahertz (GHz) (3 x 10¹¹ cycles/second). Additionally, as it is used in this disclosure, "fluid" generally refers to a liquid, a gas, or both. The term "coolant" may be used interchangeably with the term "fluid."

Like reference numerals may refer to similar or identical elements throughout the description of the figures. As shown in the drawings and described throughout this disclosure, as is traditional when referring to relative positioning on a surgical instrument, the term "proximal" refers to the end of the apparatus which is closer to the user and the term "distal" refers to the end of the apparatus which is farther away from the user. The term "clinician" refers to any medical professional (e.g., doctor, surgeon, nurse, or the like) performing a medical procedure involving the use of embodiments described herein.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto. Other elements, steps, methods and techniques that are insubstantially different from those described above and/or in the appended claims are also intended to be within the scope of the disclosure. Persons skilled in the art will understand that the systems and methods specifically described herein and illustrated in the accompanying drawings are non-limiting illustrative embodiments. The features illustrated or described in connection with one embodiment may be combined with the features of other embodiments.

The invention may be described by reference to the following numbered paragraphs:-
1. A tissue treatment system comprising:
   a treatment tool including at least one first thermal element and at least one second thermal element arranged at a distal end of the treatment tool according to an energy delivery configuration, the treatment tool being configured to deliver energy to tissue and absorb energy from tissue in a predetermined treatment zone based on the energy delivery configuration;
   a generator coupled to the treatment tool and configured to supply energy to the at least one first thermal element or the at least one second thermal element; and
   a coolant source coupled to the treatment tool and configured to supply a coolant fluid to the at least one first thermal element or the at least one second thermal element.
2. The tissue treatment system of paragraph 1, wherein the at least one first thermal element is configured to absorb energy from tissue in a first predetermined volume, and
   wherein the at least one second thermal element is configured to deliver energy to tissue in a second predetermined volume.
3. The tissue treatment system of paragraph 2, wherein the first predetermined volume is a cooling zone located in proximity to the at least one first thermal element and the second predetermined volume is a heated treatment zone located in proximity to the at least one second thermal element.
4. The tissue treatment system of paragraph 2, wherein the second predetermined volume includes at least a portion of the first predetermined volume.
5. The tissue treatment system of paragraph 1, wherein the energy delivery configuration is a cylindrical configuration wherein the at least one first thermal element is a cylindrical tubular member including a passageway and the at least one second thermal element is a cylindrical member located inside the passageway.
6. The tissue treatment system of paragraph 5, wherein the at least one second thermal element is configured to be deployed outside of the passageway.
7. The tissue treatment system of paragraph 1, wherein the energy delivery configuration is a rectangular configuration of stacked rectangular thermal elements consisting of the at least one first thermal element and the at least one second thermal element.
8. The tissue treatment system of paragraph 7, wherein the rectangular configuration consists of two first thermal elements and one second thermal element, and
   wherein the one second thermal element is bounded by the two first thermal elements.
9. The tissue treatment system of paragraph 7, wherein two first thermal elements are configured to absorb energy from tissue in a first predetermined volume, and
   wherein the one second thermal element is configured to deliver energy to tissue in a second predetermined volume.
10. The tissue treatment system of paragraph 1, wherein the treatment tool is further configured to deliver energy to tissue alternating between an activation of the at least one first thermal element and the at least one second thermal element.
11. The tissue treatment system of paragraph 1, wherein the energy delivery configuration bounds a thermal spread of the energy delivered to tissue.

## Claims

1. A tissue treatment system comprising:
a treatment tool including at least one first thermal element and at least one second thermal element arranged at a distal end of the treatment tool according to an energy delivery configuration, the treatment tool being configured to deliver energy to tissue and absorb energy from tissue in a predetermined treatment zone based on the energy delivery configuration;
a generator coupled to the treatment tool and configured to supply energy to the at least one first thermal element or the at least one second thermal element; and
a coolant source coupled to the treatment tool and configured to supply a coolant fluid to the at least one first thermal element or the at least one second thermal element.

2. The tissue treatment system of claim 1, wherein the at least one first thermal element is configured to absorb energy from tissue in a first predetermined volume, and
wherein the at least one second thermal element is configured to deliver energy to tissue in a second predetermined volume.

3. The tissue treatment system of claim 2, wherein the first predetermined volume is a cooling zone located in proximity to the at least one first thermal element and the second predetermined volume is a heated treatment zone located in proximity to the at least one second thermal element.

4. The tissue treatment system of claim 2, wherein the second predetermined volume includes at least a portion of the first predetermined volume.

5. The tissue treatment system of any preceding claim, wherein the energy delivery configuration is a cylindrical configuration wherein the at least one first thermal element is a cylindrical tubular member including a passageway and the at least one second thermal element is a cylindrical member located inside the passageway.

6. The tissue treatment system of claim 5, wherein the at least one second thermal element is configured to be deployed outside of the passageway.

7. The tissue treatment system of any preceding claim, wherein the energy delivery configuration is a rectangular configuration of stacked rectangular thermal elements consisting of the at least one first thermal element and the at least one second thermal element.

8. The tissue treatment system of claim 7, wherein the rectangular configuration consists of two first thermal elements and one second thermal element, and
wherein the one second thermal element is bounded by the two first thermal elements.

9. The tissue treatment system of claim 7, wherein two first thermal elements are configured to absorb energy from tissue in a first predetermined volume, and
wherein the one second thermal element is configured to deliver energy to tissue in a second predetermined volume.

10. The tissue treatment system of any preceding claim, wherein the treatment tool is further configured to deliver energy to tissue alternating between an activation of the at least one first thermal element and the at least one second thermal element.

11. The tissue treatment system of any preceding claim, wherein the energy delivery configuration bounds a thermal spread of the energy delivered to tissue.
